# EUROPEAN PATENT SPECIFICATION

(11) **EP 2 543 689 B1**
(45) Date of publication and mention of the grant of the patent: **10.08.2016**
(21) Application number: 11382226.6
(22) Date of filing: 04.07.2011
(51) Int. Cl.: C08G 65/00, C08G 65/26

(54) **CONTINUOUS METHOD FOR THE SYNTHESIS OF POLYOLS**
KONTINUIERLICHES VERFAHREN ZUR SYNTHESE VON POLYOLEN
PROCÉDÉ CONTINU POUR LA SYNTHÈSE DE POLYOLS

(43) Date of publication of application: 09.01.2013
(73) Proprietor: Repsol, S.A., 28045 Madrid (ES)
(72) Inventor: Almena Muñoz, Beatriz, E-28935 Móstoles - Madrid (ES); Rubio Rodríguez, Carlos, E-28935 Móstoles - Madrid (ES); Fernández Villar, Félix, E-28935 Móstoles - Madrid (ES)
(74) Representative: ABG Patentes, S.L.

(56) References cited:
- EP-A1- 1 577 334
- EP-A2- 0 090 445
- EP-A2- 1 911 787
- WO-A1-01/53381
- WO-A1-99/56874
- US-A- 5 391 722

## Description

### FIELD OF THE INVENTION

The present invention is related to a method for the continuous production of polyols.

### BACKGROUND OF THE INVENTION

Base-catalyzed oxyalkylation has been used to prepare polyoxyalkylene polyether polyols (also known simply as "polyols") for many years. In such a process, a suitable hydric low molecular weight starter or precursor molecule, such as propylene glycol or glycerine, is oxyalkylated with one or more alkylene oxides, such as ethylene oxide or propylene oxide, to form a polyoxyalkylene polyether polyol product. Strongly basic catalysts such as sodium hydroxide or potassium hydroxide are typically used in such oxyalkylations.

During base-catalyzed oxypropylation, a competing rearrangement of propylene oxide to allyl alcohol generates monofunctional species which also become oxyalkylated, producing a wide range of polyoxyalkylene monols with molecular weights ranging from that of allyl alcohol itself or its low molecular weight oxyalkylated oligomers to polyether monols of very high molecular weight. In addition to broadening the molecular weight distribution of the product, the continuous generation of monols lowers the product functionality. As the oxypropylation proceeds further, the functionality continues to decrease, and the molecular weight growth rate slows. For these reasons, the upper practical limit for base-catalyzed polyoxypropylene polyol equivalent weight is just above 2,000 Da. Even at those modest equivalent weights, the products are characterized by low actual functionality and broad molecular weight distribution.

In the early 1960's, double metal cyanide ("DMC") complexes, such as the non-stoichiometric glyme complexes of zinc hexacyanocobaltate, were found which were able to prepare polyoxypropylene polyether polyols with low monol contents, as reflected by unsaturation in the range of 0.018 to 0.020 meq/g. This represented a considerable improvement over the monol content obtainable by base catalysis.

In the 1970's, General Tire & Rubber Company, in U.S. Pat. No. 3,829,505, described the preparation of high molecular weight diols, triols etc., using double metal cyanide catalysts. However, the catalyst activity, coupled with catalyst cost and the difficulty of removing catalyst residues from the polyol product, prevented commercialization of the products.

In the 1980's, interest in such catalysts resurfaced, and improved catalysts with higher activity coupled with improved methods of catalyst removal allowed commercialization for a short time. However, the economics of the process were marginal, and in many cases, improvements expected in polymer products due to higher functionality and higher polyol molecular weight did not materialize.

In the 1990's, DMC catalysts were developed with far greater activity than was theretofore possible. Those catalysts, described for example in U.S. Pat. Nos. 5,470,813 and 5,482,908, allowed commercialization of DMC-catalyzed polyether polyols by ARCO Chemical Company.

As those skilled in the art appreciate, one drawback of DMC-catalyzed oxyalkylation is the difficulty, of using low molecular weight starters in polyether synthesis. Polyoxyalkylation of low molecular weight starters is generally sluggish, and often accompanied by catalyst deactivation. Thus, rather than employing low molecular weight starter molecules directly, oligomeric polyoxyalkylene polyether polyols are prepared in a separate process by base-catalyzed oxypropylation of a low molecular polyhydric precursor to equivalent weights in the range of 200 Da to 700 Da or higher. Further oxyalkylation to the target molecular weight takes place in the presence of DMC catalysts. However, it is known to those skilled in the art that strong bases deactivate DMC catalysts. Thus, the basic catalyst used in oligomeric polyoxyalkylene polyether polyol preparation must be removed by methods such as neutralization, adsorption, ion exchange, and the like. Several such methods require prolonged filtration of viscous polyol. The additional steps associated with catalyst removal from the oligomeric polyoxyalkylene polyether polyol can add significant process time, and thus cost, to the overall process.

It is known that basic catalysis could not provide continuous process. The solution came with a continuous process based on DMC. U.S. Pat. No. 5,777,177 and International application WO 98/03571, disclose improved processes characterized by continuous addition of starter ("CAOS") during oxyalkylation. In batch and semi-batch processes, low molecular weight polyhydric precursor, e.g., propylene glycol or dipropylene glycol, is added continuously as the polyoxyalkylation proceeds rather than all being added at the onset. As the authors disclose in WO 98/03571 that "batch processes and continuous processes are quite distinct and cannot be equated". In fact, to obtain the first practical industrial continuous process for the synthesis of polyols using DMC described in WO 98/03571 40 years had passed since the discovery of the first DMC catalysts, and almost a decade since the first commercially viable batch process.

Although the process of WO 98/03571 was a breakthrough, problems still remained to be solved. Unfortunately, when glycerine, a widely used trifunctional starter, is employed in either the batch-type continuous addition of starter process, or the continuous-type continuous addition of starter process, the DMC catalyst gradually deactivates, and often a polyether of the desired molecular weight cannot be obtained, unless process parameters are very strictly controlled. It appears that in the low molecular weight range of about 260 to 2500, where the ratio of glycerin to propylene oxide is higher than it is when making high molecular weight polyols, glycerin and other low molecular weight polyhydric precursors can act as inhibitors and stress the catalyst. Thus, a strict control of the polyhydric precursor's concentration and other low molecular weight molecules is required. In addition, these processes require very high stirring. Several procedures have been devised to overcome this problem, for example, one alternative is to acidify the polyhydric precursor prior to the reaction and thus preventing inactivation of the DMC catalyst (e.g. US 6,077,978, EP 1 911 787 and EP 1 577 334). In these processes CAOS procedure is followed; after initiating the process the reactants are mixed in the appropriate proportions at a temperature sufficient for a DMC catalyst to operate, typically above 100ºC. Although these procedures partially solve the inactivation problem, they still have drawbacks.

Another alternative solution to the problem of catalyst inactivation would be to use an acid catalyst to generate an oligomeric polyoxyalkylene polyether polyol. For example, US 5,391,722 (1995) describes a batch two-step preparation of polyols wherein the first step comprises the reaction of propylene oxide with polyhydric precursor in the presence of an acid catalyst. The oligomeric polyoxyalkylene polyether polyol obtained thereby is then reacted with further propylene oxide in the presence of DMC.

Of course, the design of a continuous version of the processes of US 5,391,722 would be highly desirable. However acid catalysis is associated with the production of cyclic impurities which lower the quality of the final product and is limited to obtaining polyols of low or medium molecular weight. (e.g. Chen et al, Macromolecules, 2003, 36, 5470-5481 or US 2004/0010165 in the name of Bayer Polymers LLC). In addition, when produced under continuous conditions, the oligomeric polyoxyalkylene polyether polyols obtained display broad molecular weight distributions which in turn should provide polyols with broad molecular weight distributions. Therefore, since 1995 no continuous process using acid catalyst to generate the initiator has been described.

### SUMMARY OF THE INVENTION

After extensive investigation, the inventors have developed a continuous process for the preparation of polyols which starts with the continuous production of an oligomeric polyoxyalkylene polyether polyol under acid catalysis. The process of the invention provides final polyols having comparable physic-chemical characteristics (e.g. viscosity or insaturation) to its batch version despite the broad molecular weight distribution of the intermediate oligomeric polyoxyalkylene polyether polyol. Surprisingly the continuous process of the invention is devoid of the problems resulting from the presence of cyclic impurities described in bibliography. The inventors have also found that the process is more robust than other known continuous processes and DMC catalyst inactivation is significantly reduced.

Thus it is an aspect of the present invention to provide a process for the preparation of polyoxyalkylene polyether polyols which comprises:
(i) continuously reacting at least one polyhydric precursor and at least one alkylene oxide in the presence of at least one acid catalyst and, optionally in the presence of a double metal cyanide complex catalyst, at a temperature below that necessary to activate said double metal cyanide complex catalyst;
(ii) continuously removing the mixture comprising the oligomeric polyoxyalkylene polyether polyol formed in the previous step, adding double metal cyanide complex catalyst if required, and raising the temperature to that needed to activate the double metal cyanide complex catalyst while adding further alkylene oxide or mixtures of alkylene oxides; and
(iii) continuously removing the final polyoxyalkylene polyether polyol formed.

Polyols are the starting materials for the synthesis of polyurethanes. Thus a further aspect of the invention is a method for the synthesis of polyurethanes which comprises the reaction between a polyol and an isocyanate wherein said polyol has been prepared by the continuous process of the invention, and the polyurethanes obtained. Said method can be obtained following the conditions generally known in the art, such as EP2256141, WO10020367A2, WO1001660, EP0010842, EP0035615 or EP0273099.

A further aspect described herein is an oligomeric polyoxyalkylene polyether polyol obtainable after step (i) of the process of the invention. This intermediate product does not show the problems of acid-generated oligomeric polyoxyalkylene polyether polyol known in the prior art.

A further aspect of the invention is a polyoxyalkylene polyether polyol obtainable after step (ii) of the process of the invention. The generation of these final polyoxyalkylene polyether polyol from the unique acid-generated intermediate oligomers provides a product which differs from those known in the art.

### DETAILED DESCRIPTION OF THE INVENTION

After setting up the conditions necessary to initiate the continuous process, the process of the invention begins by preparing an oligomeric polyoxyalkylene polyether polyol under acid catalysis. The conditions, namely the temperature, in this step are such that the DMC, although it may be present, remains inactive. In a preferred embodiment the temperature in step (i) is below 80ºC. Typically, the temperature in this step is between 0 and 70ºC, preferably between 30 and 60ºC. In a further embodiment of the invention the temperature is between 45 and 55ºC, more particurlarly between 45 and 50ºC. Said temperatures prevent activation of DMC, but also the decomposition of the acid catalyst.

The conditions in the second step are such that the DMC becomes active and thus the temperature in step (ii) is typically raised above 80ºC, although any temperature sufficient to activate the catalyst is within the scope of the invention. In an embodiment of the invention the temperature in step (ii) is between 100 and 200ºC, preferably between 100 and 160ºC. In a further embodiment the temperature is between 120 and 150ºC, more particularly between 130 and 145ºC.

Thus, the DMC may be added to the reaction mixture in the first step. In step (ii) DMC catalyst is necessary to proceed with the reaction and further increase of the molecular weight of the oligomeric polyoxyalkylene polyether polyol to obtain the final polyoxyalkylene polyether polyol. Therefore, in this second step (ii) DMC catalyst is added if necessary. If DMC catalyst has been added in the first step (i), it may be necessary to add further DMC catalyst in the second step (ii). On the other hand, the amount of DMC catalyst added, if any, in the first step (i) may be sufficient to proceed with the second step (ii) without adding further DMC. The only requirement is that sufficient amount of DMC catalyst is present in the mixture for the second step, regardless of whether it has been added on step (i), step (ii) or both. In an embodiment of the invention, DMC catalyst is added on step (i).

The polyhydric precursor may be any low molecular weight organic molecule having two or more reactive hydrogens. Typical polyhydric precursors are ethylene glycol; diethylene glycol; triethylene glycol; propylene glycol; dipropylene glycol; tripropylene glycol; 1,2-, 1,3-, and 1,4-butylene glycols; neopentyl glycol; glycerine, trimethylolpropane; triethylolpropane; pentaerythritol, alpha-methylglucoside; hydroxy-methyl-, hydroxyethyl-, and hydroxypropylglucosides; sorbitol, mannitol; sucrose; tetrakis [2-hydroxyethyland 2-hydroxypropyl]ethylene diamines; and other commonly used starters. Also suitable are monofunctional starters such as methanol, ethanol, 1-propanol, 2-propanol, n-butanol, 2-butanol, 2-ethylhexanol, and the like, as well as phenol, catechol, 4,4'-dihydroxybiphenyl, or 4,4'-dihydroxydiphenylmethane. According to an embodiment of the invention said polyhydric precursor is glycerine. And one of the advantages of the invention is that the process does not require dehydrating glycerine prior to use.

Although virtually any organic or inorganic acid may be used in the process of the present invention, useful acids include, but are not limited to, the Lewis acids. Examples of Lewis acid are those selected from the group consisting of antimony pentafluoride, antimony pentachloride, trifluoromethane sulfonic acid, boron trifluoride etherate, tetrafluoroboric acid and combinations thereof. In a particular embodiment the acid is tetrafluoroboric acid.

The amount of acid added in the continuous process of the invention is sufficient to allow acid catalysis in the step (i), and not as much as to interfere in the DMC catalysis of step (ii). Exemplary amounts 100 ppm to 5,000 ppm, 500 to 4,000 ppm, or 1,000 to 3,000 ppm. In an embodiment of the invention the acid is added as aqueous solution.

The continuous process of the invention allows generation of polyols of a wide range of molecular weights having good to acceptable molecular weight distributions. In an embodiment of the invention the final polyoxyalkylene polyether polyol formed is above 1,500 Da.

In an embodiment of the invention, the oligomeric polyoxyalkylene polyether polyol formed in step (i) has a molecular weight above 300. In a further embodiment between 350 and 1,000 Da, preferably between 400 and 800 Da.

The alkylene oxides useful in the inventive process include, but are not limited to, ethylene oxide, propylene oxide, oxetane, 1,2- and 2,3-butylene oxide, isobutylene oxide, epichlorohydrin, cyclohexene oxide, styrene oxide, and the higher alkylene oxides such as the C₅ - C₃₀ alpha -alkylene oxides. Propylene oxide alone or mixtures of propylene oxide with ethylene oxide or another alkylene oxide are preferred. Other polymerizable monomers may be used as well, e.g., anhydrides and other monomers as disclosed in U.S. Pat. Nos. 3,404,109, 3,538,043 and 5,145,883, the contents of which are herein incorporated in their entireties by reference thereto.

The process of the present invention may employ any double metal cyanide (DMC) catalyst. Double metal cyanide complex catalysts are non-stoichiometric complexes of a low molecular weight organic complexing agent and optionally other complexing agents with a double metal cyanide salt, e.g. zinc hexacyanocobaltate. Suitable DMC catalysts are known to those skilled in the art. Exemplary DMC catalysts include those suitable for preparation of low unsaturation polyoxyalkylene polyether polyols, such as disclosed in U.S. Pat. Nos. 3,427,256; 3,427,334; 3,427,335; 3,829,505; 4,472,560; 4,477,589; and 5,158,922, the contents of which are incorporated herein in their entireties by reference thereto. The DMC catalysts more preferred in the process of the present invention are those capable of preparing "ultra-low" unsaturation polyether polyols. Such catalysts are disclosed in U.S. Pat. Nos. 5,470,813 and 5,482,908, 5,545,601, 6,689,710 and U.S. Published Patent Application No. 2004-0044240-A1, the contents of which are herein incorporated in their entireties by reference thereto. Particularly preferred in the inventive process are those zinc hexacyanocobaltate catalysts prepared by the methods described in U.S. Pat. No. 5,482,908.

In an embodiment of the invention, the DMC catalyst is that prepared according to the process described in the European patent application EP11382156.5 filed 17 May 2011 with the tittle PROCESS FOR PREPARING HIGHLY ACTIVE DOUBLE METAL CYANIDE CATALYSTS AND THEIR USE IN THE SYNTHESIS OF POLYETHER POLYOLS.

The DMC catalyst concentration is chosen so as to ensure good control of the polyoxyalkylation reaction under the given reaction conditions. The catalyst concentration is preferably in the range from 0.0005 % wt. to 0.1 % wt., more preferably in the range from 0.001 % wt. to 0.1 % wt., most preferably in the range from 0.001 to 0.01 % wt., based on the amount of polyol to be produced. The DMC catalyst may be present in the process of the present invention in an amount ranging between any combination of these values, inclusive of the recited values.

The process of the invention can take place using any suitable installations for continuous processes. In the different steps of the invention one or more reactors can be used. Said reactors can be for example tubular or continuous stirred tank reactors (CSTR).

In an embodiment of the invention the oligomeric polyoxyalkylene polyether polyol is produced in a first CSTR, from which the mixture containing the oligomeric polyoxyalkylene polyether polyol, the acid catalyst and the DMC catalyst, is continuously transfered to a second CSTR wherein the alkylene oxide is continuously added so as to produce the final polyoxyalkylene polyether polyol of the target molecular weight. According to a preferred embodiment, the size of the first reactor wherein the oligomeric polyoxyalkylene polyether polyol is generated under acid conditions is smaller than the said second reactor. According to this embodiment the reactor is 1.5 to 10 times smaller, preferably between 1.5 to 5.

Alkylene oxide together with starter or low molecular weight oxyalkylation product may be added at various points along the reactor. In an embodiment of the invention a stream feed comprising the oligomeric polyoxyalkylene polyether polyol, the acid catalyst and the DMC catalyst, is premixed with the feed stream comprising the alkylene oxide prior, and then the mixture of all four materials is added to the reactor.

The process of the invention is far more productive than that described in US 5,391,722. According to an embodiment of the invention the productivity is 1.5 to 5 times higher than the productivity in a semibatch process, more particularly between 2 to 4 times higher as measured in Kg/h under the same conditions (as measured in the examples below).

The process of the invention may be further completed by "cooking-out" the final polyoxyalkylene polyether polyol without adding further alkylene oxide. This cooking can be effected in a further reactor. The resulting final polyoxyalkylene polyether polyol can be filtered and/or purified according to procedures known to the skilled person.

As used herein, the term "continuous" means a mode of addition of a relevant reactant in such a manner so as to maintain an effective concentration of the reactant substantially continuously. Continuous starter addition, for example, may be truly continuous, or may be in relatively closely spaced increments. It would not detract from the present process to incrementally add a reactant in such a manner that the added material's concentration decreases to essentially zero for some time prior to the next incremental addition. Incremental addition of reactant which does not substantially affect the nature of the product is still "continuous" as that term is used herein.

### EXAMPLES

### Example 1

### Semibatch process (comparative example)

A 2 liter capacity Büchi reactor loaded with 200 g of glycerin is provided. It is heated at 50ºC and stirring starts, and once the temperature is stabilized, 3 g of acid catalyst (HBF4, 50 % wt. aqueous solution) are added. Then, a propylene oxide flow is fed into the reactor (mean flow rate = 6.4 g/min), until 1300 g of such oxide is loaded. Upon unloading the reactor, an oligomeric polyoxyalkylene polyether polyol of 700 Da is obtained.

The oligomeric polyoxyalkylene polyether polyol synthesized in this first stage is used as raw material to produce a polyol with a molecular weight of 3,500 Da. The process followed is described below:

300 g of the acid oligomeric polyoxyalkylene polyether polyol with a molecular weight of 700, previously synthesized with acid catalysis, are added in a 2 liter Büchi reactor. It is stirred and heated at 140ºC in inert atmosphere. A vacuum with N₂ bubbling is generated to remove the humidity from the polyether for the time necessary to achieve a humidity less than 100 ppm. 30 ppm of DMC catalyst are then added, and it is stirred for another 10 minutes under vacuum without the need of N₂ bubbling.

45 g (15%) of propylene oxide are then added to activate the catalyst. After a period of time, the pressure drops abruptly to the initial value, which will be the sign that the catalyst has already been activated. The continuous loading of the propylene oxide/ethylene oxide mixture is then performed until reaching the desired molecular weight. The propylene oxide is fed at 140ºC and without the absolute pressure exceeding 1.5 kg/cm². Once all the PO/EO has been added, it is left to post-react until constant pressure is reached. Finally, the residual monomers are removed under a vacuum with N₂ bubbling for 30 minutes. The final polyol obtained (3,500 Da) has the following properties: hydroxyl number = 48 mg KOH/g, viscosity = 782 cP, polydispersity = 1.22, unsaturation = 0.008 meq/g.

The total time invested in the preparation of the polyol in the described stages was 3.6 hours for obtaining 1 Kg of polyol. Therefore, the productivity of the process is 0.3 Kg/h. To provide an appropriate comparison, it has been supposed to obtain this value that the total quantity of oligomeric polyoxyalkylene polyether polyol synthesized in the first stage (1500 g) has been used to produce 5 batches (7500 g) of polyol, with the cycle times of the lab installation.

### Example 2

### Starting up the installation for continuous process

The continuous process is started up by following techniques known by the person skilled in the art. In this case, an installation with three CSTR reactors, the first of them with a capacity of 0.5 liters, and the other two with a capacity of 1 liter each, is provided.

For starting the process, 67 g of glycerin and 2 g of acid catalyst (HBF4, 50 % wt. aqueous solution) are loaded in reactor 1. Stirring starts and it is heated to 50ºC. Once this temperature is reached, the continuous addition of a propylene oxide with a flow rate of 4 g/min is added until reaching a molecular weight of 700 Da.

200 g of the oligomeric polyoxyalkylene polyether polyol produced in reactor 1 are transferred to reactor 2; it is heated at 140ºC under stirring and a vacuum time of 1 hour at 15 mbar and with nitrogen bubbling is maintained. The DMC catalyst (0.03 g) is then added and the activation is performed with a propylene oxide load of 30 g. An abrupt lowering of pressure indicates that the DMC catalyst has been suitably activated. At this time, an oxide mixture (propylene oxide/ethylene oxide, 7:1) is fed at a continuous flow rate of 8 g/min until reaching a product molecular weight of 3,500 Da. The same procedure is carried out in the reactor 3 to activate the system.

Once these steps are performed, the installation is prepared to start the continuous mode operation. The process used for the continuous manufacture of a polyol of 3,500 Da using an acid oligomeric polyoxyalkylene polyether polyol as a precursor, which is also synthesized continuously, is indicated in the following examples.

### Example 3

### Continuous process of the invention

Two different feed lines are used in reactor 1, one for the propylene oxide and another additional line for the mixture of glycerin/acid catalyst (HBF4)/DMC catalyst. The reaction heat generated during polyoxyalkylation is removed by means of an internal cooling circuit. The operation is carried out under stirring at a temperature of 50ºC and at a pressure of 4 barg. Once these conditions are fixed, the pumps of the two input flows are simultaneously operated. The supply flow rates are fixed to provide a residence time of 225 minutes in reactor 1. The ratio of the primer (glycerin) and propylene oxide is adjusted to produce an oligomeric polyoxyalkylene polyether polyol of 700 Da. The catalyst concentrations used are: 2,000 ppm of HBF4 and 150 ppm of DMC catalyst with respect to the oligomeric polyoxyalkylene polyether polyol produced.

The outlet flow of reactor 1 is directed continuously to reactor 2 which in turn is fed another flow with the alkylene oxide mixture (propylene oxide/ethylene oxide, 7:1). In this reactor, the reaction heat generated during polyoxyalkylation is also removed by means of an internal cooling circuit. The operation is carried out under stirring at 140ºC and 4 barg. The pump of the alkylene oxide supply flow is operated at the same time the operation in reactor 1 starts. The flow rates are fixed to provide a residence time of 90 minutes in reactor 2. The ratio between the oligomeric polyoxyalkylene polyether polyol and alkylene oxides is adjusted to produce a final polyoxyalkylene polyether polyol of 3,500 Da.

After keeping the system operating for 12 hours, a final polyoxyalkylene polyether polyol of 3,500 Da is obtained having physic-chemical properties similar to those of the prior art.

Taking the operation flow rates into account, a final polyoxyalkylene polyether polyol generation rate of 0.7 Kg/h is obtained in the outlet flow. Therefore, the productivity associated with this process is at least 2 times greater than that obtained in Comparative Example 1.

### Example 4

### Continuous process of the invention

An oligomeric polyoxyalkylene polyether polyol of 700 Da is continuously produced using reactor 1 of Example 3 in the same conditions (temperature, pressure, stirring, residence time and catalyst addition).

The outlet flow of reactor 1 is directed continuously to reactor 2 which in turn is fed another flow with the alkylene oxide mixture (propylene oxide/ethylene oxide, 7:1). In this reactor, the reaction heat generated during polyoxyalkylation is also removed by means of an internal cooling circuit. The operation is carried out under stirring at 140ºC and 4 barg. The pump of the alkylene oxide supply flow is operated at the same time the operation in reactor 1 starts. The flow rates are fixed to provide a residence time of 120 minutes in reactor 2. The ratio between the oligomeric polyoxyalkylene polyether polyol and alkylene oxides is adjusted to produce a polyol of 2,660 Da.

The outlet flow of reactor 2 is directed continuously to reactor 3 which in turn is fed another flow with the alkylene oxide mixture (propylene oxide/ethylene oxide, 7:1). In this reactor, the reaction heat generated during polyoxyalkylation is also removed by means of an internal cooling circuit. The operation is carried out under stirring at 140ºC and 4 barg. The pump of the alkylene oxide supply flow is operated at the same time the operation in reactors 1 and 2 starts. The flow rates are fixed to provide a residence time of 90 minutes in reactor 3. The ratio between the polyoxyalkylene polyether polyol of 2,660 Da and alkylene oxides is adjusted to produce a final polyoxyalkylene polyether polyol of 3,500 Da.

After keeping the system operating for 12 hours, a polyol of 3,500 Da is obtained having physic-chemical properties similar to those of the prior art.

Taking the operation flow rates into account, a final polyoxyalkylene polyether polyol generation rate of 0.7 Kg/h is obtained in the outlet flow. Therefore, the productivity associated with this process is at least 2 times greater than that obtained in Example 1 and the properties of the final product are equivalent.

### Example 5

### Continuous process of the invention

Two different feed lines are used in reactor 1, one for the propylene oxide and another additional line for the mixture of glycerin/acid catalyst (HBF4). The reaction heat generated during polyoxyalkylation is removed by means of an internal cooling circuit. The operation is carried out under stirring at a temperature of 50ºC and at a pressure of 4 barg. Once these conditions are fixed, the pumps of the two input flows are simultaneously operated. The supply flow rates are fixed to provide a residence time of 225 minutes in reactor 1. The ratio of glycerin and propylene oxide is adjusted to produce an oligomeric polyoxyalkylene polyether polyol of 700 Da. The catalyst concentration used is 2,000 ppm of HBF4 with respect to this product.

The outlet flow of reactor 1 is directed continuously to reactor 2, where two more feed lines are used, one for the alkylene oxide mixture (propylene oxide/ethylene oxide, 7:1), and another recycle line to add the DMC catalyst (30 ppm with respect to the final polyoxyalkylene polyether polyol). In this reactor, the reaction heat generated during polyoxyalkylation is also removed by means of an internal cooling circuit. The operation is carried out under stirring at 140ºC and 4 barg. The pump of the alkylene oxide supply flow is operated at the same time the operation in reactor 1 starts. The flow rates are fixed to provide a residence time of 120 minutes in reactor 2. The ratio between the oligomeric polyoxyalkylene polyether polyol and alkylene oxides is adjusted to produce a polyoxyalkylene polyether polyol of 2,660 Da.

The outlet flow of reactor 2 is directed continuously to reactor 3 which in turn is fed another flow with the alkylene oxide mixture (propylene oxide/ethylene oxide, 7:1). In this reactor, the reaction heat generated during polyoxyalkylation is also removed by means of an internal cooling circuit. The operation is carried out under stirring at 140ºC and 4 barg. The pump of the alkylene oxide supply flow is operated at the same time the operation in reactors 1 and 2 starts. The flow rates are fixed to provide a residence time of 90 minutes in reactor 3. The ratio between the polyoxyalkylene polyether polyol of 2,660 Da and alkylene oxides is adjusted to produce a final polyoxyalkylene polyether polyol of 3,500 Da.

After keeping the system operating for 12 hours, a final polyoxyalkylene polyether polyol of 3,500 Da is obtained, having physic-chemical properties similar to those of the prior art.

Taking the operation flow rates into account, a final polyoxyalkylene polyether polyol generation rate of 0.7 Kg/h is obtained in the outlet flow. Therefore, the productivity associated with this process is at least 2 times greater than that obtained in Example 1 and the properties of the final product are equivalent.

## Claims

1. A continuous process for the preparation of polyoxyalkylene polyether polyols which comprises:
(i) continuously reacting at least one polyhydric precursor and at least one alkylene oxide in the presence of at least one acid catalyst and, optionally in the presence of a double metal cyanide complex catalyst, at a temperature below that necessary to activate said double metal cyanide complex catalyst;
(ii) continuously removing the mixture comprising the oligomeric polyoxyalkylene polyether polyol formed in the previous step, adding to said mixture double metal cyanide complex catalyst if required, and raising the temperature to that needed to activate the double metal cyanide complex catalyst while adding further alkylene oxide or mixtures of alkylene oxides;
and
(iii) continuously removing the final polyoxyalkylene polyether polyol formed,
with the proviso that, whether a double metal cyanide complex catalyst is added in step (i), step (ii) or both, sufficient amount of double metal cyanide complex catalyst is present in the mixture for obtaining the final polyoxyalkylene polyether polyol from the oligomeric polyoxyalkylene polyether polyol.

2. Process according to claim 1, wherein the temperature in step (i) is below 80°C.

3. Process according to claim 2, wherein the temperature in step (i) is between 0 and 70°C.

4. Process according to claims 1 to 3, wherein the temperature in step (i) is between 30 and 60°C.

5. Process according to any of claims 1 to 4, wherein the temperature in step (ii) is above 80°C.

6. Process according to claim 5, wherein the temperature in step (ii) is between 100 and 200°C.

7. Process according to claim 6, wherein the temperature in step (ii) is between 100 and 160°C.

8. Process according to any of claims 1 to 7 wherein said polyhydric precursor is glycerine.

9. Process according to any of claims 1 to 8, wherein said acid catalyst is selected from the group consisting of antimony pentafluoride, antimony pentachloride, trifluoromethane sufonic acid, boron trifluoride etherate, tetrafluoroboric acid, and combinations thereof.

10. Process according to any of claims 1 to 9, wherein the oligomeric polyoxyalkylene polyether polyol formed in step (i) has a molecular weight above 300.

11. Process according to claim 10, wherein the oligomeric polyoxyalkylene polyether polyol formed in step (i) has a molecular weight comprised between 350 and 1,000 Da.

12. Process according to claim 11, wherein the oligomeric polyoxyalkylene polyether polyol formed in step (i) has a molecular weight comprised between 400 and 800 Da.

13. Process according to any of claims 1 to 12, wherein the molecular weight of the final polyoxyalkylene polyether polyol formed is above 1,500 Da.

14. A polyoxyalkylene polyether polyol obtainable after step (ii) of the process defined in claim 1.

15. A method for the synthesis of polyurethanes which comprises the reaction between a polyoxyalkylene polyether polyol obtainable by the continuous process defined in any of claims 1 to 13 and an isocyanate.

16. A polyurethane obtainable by the method defined in claim 15.

## Patentansprüche

1. Kontinuierliches Verfahren zur Herstellung von Polyoxyalkylenpolyetherpolyolen, umfassend:
(i) kontinuierliches Umsetzen von zumindest einem polyhydrischen Vorläufer und zumindest einem Alkylenoxid in Gegenwart von zumindest einem Säurekatalysator und gegebenenfalls in Gegenwart eines Doppelmetallcyanidkomplex-Katalysators bei einer Temperatur unterhalb derjenigen, die zur Aktivierung dieses Doppelmetallcyanidkomplex-Katalysators erforderlich ist;
(ii) kontinuierliches Entfernen der Mischung, die das im vorherigen Schritt gebildete oligomere Polyoxyalkylenpolyetherpolyol umfasst, Zugabe von Doppelmetallcyanidkomplex-Katalysator zu dieser Mischung, falls erforderlich, und Erhöhung der Temperatur auf eine solche, die zur Aktivierung des Doppelmetallcyanidkomplex-Katalysators erforderlich ist, während weiteres Alkylenoxid oder Mischungen von Alkylenoxiden zugegeben wird/werden; und
(iii) kontinuierliches Entfernen des gebildeten endgültigen Polyoxyalkylenpolyetherpolyols,
mit der Massgabe, dass, wenn ein Doppelmetallcyanidkomplex-Katalysator in Schritt (i), Schritt (ii) oder beiden zugegeben wird, eine ausreichende Menge an Doppelmetallcyanidkomplex-Katalysator in der Mischung vorhanden ist, um das endgültige Polyoxyalkylenpolyetherpolyol aus dem oligomeren Polyoxyalkylenpolyetherpolyol zu erhalten.

2. Verfahren gemäss Anspruch 1, wobei die Temperatur in Schritt (i) unterhalb 80°C liegt.

3. Verfahren gemäss Anspruch 2, wobei die Temperatur in Schritt (i) zwischen 0 und 70°C liegt.

4. Verfahren gemäss Ansprüchen 1 bis 3, wobei die Temperatur in Schritt (i) zwischen 30 und 60°C liegt.

5. Verfahren gemäss irgendeinem der Ansprüche 1 bis 4, wobei die Temperatur in Schritt (ii) oberhalb 80°C liegt.

6. Verfahren gemäss Anspruch 5, wobei die Temperatur in Schritt (ii) zwischen 100 und 200°C liegt.

7. Verfahren gemäss Anspruch 6, wobei die Temperatur in Schritt (ii) zwischen 100 und 160°C liegt.

8. Verfahren gemäss irgendeinem der Ansprüche 1 bis 7, wobei der polyhydrische Vorläufer Glycerin ist.

9. Verfahren gemäss irgendeinem der Ansprüche 1 bis 8, wobei der Säurekatalysator aus der Gruppe bestehend aus Antimonpentafluorid, Antimonpentachlorid, Trifluormethansulfonsäure, Bortrifluoridetherat, Tetrafluorborsäure und Kombinationen davon ausgewählt ist.

10. Verfahren gemäss irgendeinem der Ansprüche 1 bis 9, wobei das in Schritt (i) gebildete oligomere Polyoxyalkylenpolyetherpolyol ein Molekulargewicht oberhalb 300 aufweist.

11. Verfahren gemäss Anspruch 10, wobei das in Schritt (i) gebildete oligomere Polyoxyalkylenpolyetherpolyol ein Molekulargewicht zwischen 350 und 1.000 Da aufweist.

12. Verfahren gemäss Anspruch 11, wobei das in Schritt (i) gebildete oligomere Polyoxyalkylenpolyetherpolyol ein Molekulargewicht zwischen 400 und 800 Da aufweist.

13. Verfahren gemäss irgendeinem der Ansprüche 1 bis 12, wobei das Molekulargewicht des gebildeten endgültigen Polyoxyalkylenpolyetherpolyols oberhalb 1.500 Da liegt.

14. Polyoxyalkylenpolyetherpolyol, erhältlich nach Schritt (ii) des in Anspruch 1 definierten Verfahrens.

15. Verfahren zur Synthese von Polyurethanen, das die Umsetzung zwischen einem Polyoxyalkylenpolyetherpolyol, das durch das in irgendeinem der Ansprüche 1 bis 13 definierte kontinuierliche Verfahren erhältlich ist, und einem Isocyanat umfasst.

16. Polyurethan, erhältlich nach dem in Anspruch 15 definierten Verfahren.

## Revendications

1. Procédé continu de préparation de polyoxyalkylène polyéther-polyols qui comprend:
(i) la mise en réaction en continu d'au moins un précurseur polyhydrique et d'au moins un oxyde d'alkylène en présence d'au moins un catalyseur acide et, facultativement, en présence d'un catalyseur à base de complexe de cyanure bimétallique, à une température inférieure à celle nécessaire pour activer ledit catalyseur à base de complexe de cyanure bimétallique ;
(ii) le retrait en continu du mélange comprenant le polyoxyalkylène polyéther-polyol oligomérique formé à l'étape précédente, l'ajout dudit mélange audit mélange du catalyseur à base de complexe de cyanure bimétallique si nécessaire, et l'augmentation de la température à celle requise pour activer le catalyseur à base de complexe de cyanure bimétallique tout en ajoutant une quantité supplémentaire d'oxydes d'alkylène ou de mélanges d'oxydes d'alkylène ; et
(iii) le retrait en continu du polyoxyalkylène polyéther-polyol final ainsi formé, à la condition que, qu'un catalyseur à base de complexe de cyanure bimétallique soit ajouté à l'étape (i), l'étape (ii) ou aux deux étapes, une quantité suffisante de catalyseur à base de complexe de cyanure bimétallique soit présente dans le mélange afin d'obtenir le polyoxyalkylène polyéther-polyol final à partir du polyoxyalkylène polyéther-polyol oligomérique.

2. Procédé selon la revendication 1, dans lequel la température à l'étape (i) est inférieure à 80 °C.

3. Procédé selon la revendication 2, dans lequel la température à l'étape (i) est entre 0 et 70 °C.

4. Procédé selon les revendications 1 à 3, dans lequel la température à l'étape (i) est entre 30 et 60 °C.

5. Procédé selon l'une quelconque des revendications 1 à 4, dans lequel la température à l'étape (ii) est supérieure à 80 °C.

6. Procédé selon la revendication 5, dans lequel la température à l'étape (ii) est entre 100 et 200 °C.

7. Procédé selon la revendication 6, dans lequel la température à l'étape (ii) est entre 100 et 160 °C.

8. Procédé selon l'une quelconque des revendications 1 à 7, dans lequel ledit précurseur polyhydrique est de la glycérine.

9. Procédé selon l'une quelconque des revendications 1 à 8, dans lequel ledit catalyseur acide est sélectionné parmi le groupe constitué de pentafluorure d'antimoine, de pentachlorure d'antimoine, d'acide sulfonique trifluorométhane, d'éthérate de trifluorure de bore, d'acide tétrafluoroborique, et de combinaisons de ces derniers.

10. Procédé selon l'une quelconque des revendications 1 à 9, dans lequel le polyoxyalkylène polyéther-polyol oligomérique formé à l'étape (i) présente un poids moléculaire supérieur à 300.

11. Procédé selon la revendication 10, dans lequel le polyoxyalkylène polyéther-polyol oligomérique formé à l'étape (i) présente un poids moléculaire compris entre 350 et 1000 Da.

12. Procédé selon la revendication 11, dans lequel le polyoxyalkylène polyéther-polyol oligomérique formé à l'étape (i) présente un poids moléculaire compris entre 400 et 800 Da.

13. Procédé selon l'une quelconque des revendications 1 à 12, dans lequel le poids moléculaire du polyoxyalkylène polyéther-polyol final ainsi formé est supérieur à 1500 Da.

14. Polyoxyalkylène polyéther-polyol susceptible d'être obtenu après l'étape (ii) du procédé défini dans revendication 1.

15. Procédé de synthèse de polyuréthanes qui comprend la réaction entre un polyoxyalkylène polyéther-polyol susceptible d'être obtenu à l'aide du procédé continu défini dans l'une quelconque des revendications 1 à 13 et d'un isocyanate.

16. Polyuréthane susceptible d'être obtenu à l'aide du procédé défini selon la revendication 15.
